# EUROPEAN PATENT APPLICATION

(11) **EP 1 438 979 A1**
(43) Date of publication of application: **21.07.2004**
(21) Application number: 02080640.2
(22) Date of filing: 19.12.2002
(51) Int. Cl.: A61M 5/32

(54) **Disposable hypodermic syringe**

(71) Applicant: Brocks, Henrik, 2700 Bronshoj (DK); Brocks, Regina, 2700 Bronshoj (DK)
(72) Inventor: Brocks, Henrik, 2700 Bronshoj (DK); Brocks, Regina, 2700 Bronshoj (DK)
(74) Representative: Elmeros, Claus

(57) **Abstract**

The present invention relates to a disposable hypodermic syringe the needle (31) of which being retained by fixation means (16). When the syringe has been used, the needle is retracted into the syringe plunger barrel (21), effectively preventing reuse and exposure of the used and possibly disease carrying needle.

## Description

### Field of the invention

The present invention relates to a disposable hypodermic syringe, having a retractable needle.

### Background of the invention

Working with hypodermic syringes involves in addition to the risk of immediate injuries due to sticking oneself, the much more dangerous risk of being infected with diseases carried by used syringe needles, eg diseases transmitted by blood. Several of these diseases, eg AIDS and hepatitis, are very serious, and may often cause death.

In hospitals and clinics, where hypodermic syringes are used everyday, the staff are used to handle such syringes, know the danger, and have fixed procedures on the handling and disposal of used syringes. The risk of catching a disease from a hypodermic needle is thus significantly greater in environments where syringes are exposed to people not knowing about the existence of the syringes, or not knowing about the danger they make out. Such environments comprise eg parks or other areas open to the public, where eg drug addicts may use trashcans or shrubbery for disposal of their syringes. In such environments the used syringes may be exposed to eg garbage collectors, children and animals. Other risky situations comprise eg police officers having to body search eg drug addicts, etc., and drug addicts using used syringes.

One way to counteract the above-described risks may be to allow the needle to be retracted into the syringe barrel after use, as disclosed by several patents. Such inventions comprise eg syringes with manually retractable needles, eg as disclosed in US 4,650,468 and syringes having a spring for automatic retraction of the needle together with a kind of button to release the spring and retract the needle, eg as disclosed in US 6,183,440.

Other solutions allowing the needle to be retracted into the syringe barrel comprise a spring-loaded needle being released when the plunger is fully depressed into the syringe barrel. Several patents disclose such syringes, eg DK 156414 and US 5,843,034, both having a needle mount being retained by fins positioned on the inside surface of the syringe barrel, and released when the plunger is fully depressed and thereby forces the fins outwards. European patent applications EP 1 166 809, EP 1 166 810, EP 1 177 803, EP 1 184 048 and EP 1 192 967 all disclose syringes having a cylindrical cutter, which cuts a hole in the bottom of the plunger and cuts the needle mount free, upon full depression of the plunger.

Disadvantages of the above-mentioned solutions comprise relatively many and advanced parts, and especially the European patent applications mentioned requires use of an unexpectedly excessive force, or an extremely sharp, and thereby expensive to manufacture, cylindrical cutter.

An alternative solution is disclosed by Danish utility model DK 1998 00421, which describes a hypodermic syringe having a spring-loaded needle mounted in a ball, retained by a ball catch. When its plunger, comprising a hollow barrel whose stopper end is sealed with a foil diaphragm, is fully depressed, the foil diaphragm is penetrated by the rear end of the needle, which protrudes from the ball, and at the same time the ball catch releases the needle mount and needle, which is by the spring pushed into the hollow plunger, where it remains unexposed. Because of the use of a ball catch to retain and release the needle mount, the retraction of the needle after use is easy for the user to set off.

A very serious disadvantage of the above-described utility model is the risk of injecting air bubbles into a vein. Unlike most hypodermic syringe embodiments which eliminate this risk in different ways, the above-described utility model makes it very difficult to prevent air bubbles because of the protruding rear-end of the needle to break the foil diaphragm, and because of the ball-shaped needle mount.

One object of the present invention is to provide a disposable hypodermic syringe with an automatically retractable needle, which is easy to release, cost-effective and simple, and which eliminates the risk of injecting air bubbles into the blood stream.

### Summary of the invention

The present invention relates to a disposable hypodermic syringe comprising a syringe body (1), comprising fixation means (16) for releasably retaining a spring loaded hypodermic needle (31) whose internal end is mounted in a needle mount (32), and a plunger (2); characterized in that said needle mount (32) has a cavity (34) extending from its internal end.

According to the present invention, an advantageous way of preventing used and possibly disease carrying needles from single-use syringes from being exposed to eg kids playing outside, garbage collectors, nurses, police officers, etc, has been obtained. With the present invention is prevention of reuse of such syringes furthermore achieved.

According to the invention, the needle is firmly retained by the fixation means during use, and is released when the plunger is fully depressed into the syringe body. As the needle is spring loaded, it is subsequently irreversibly retracted into the syringe body, where it remains unexposed, and thereby prevents people from being hurt by it.

According to the present invention, an advantageous achievement has been obtained by making a cavity in the needle mount. Thereby it is easy for the user of the syringe to collect and push all air bubbles out of the fluid medicine or other liquid before injection. As injection of air bubbles into the blood stream of a living person may cause serious danger and even death, it is very important to be able to prevent this from happening.

When said fixation means (16) comprises a latch (17), a locking ring (5) and a recess (18), an advantageous embodiment of the present invention has been obtained.

According to this preferred embodiment of the invention, the latch is prevented from releasing the needle mount because of the locking ring. When the locking ring is pushed down into the recess, the latch is able to open. Thereby an advantageous, simple and easy-to-use way of releasing the needle mount has been obtained.

When said plunger (2) comprises a hollow barrel (21), an advantageous embodiment of the present invention has been obtained.

According to this preferred embodiment of the invention, the plunger barrel is hollow to allow the hypodermic needle and the needle mount to be retracted into the plunger.

When the internal end of said hollow barrel (21) of said plunger (2) is closed with a foil diaphragm (24), an advantageous embodiment of the present invention has been obtained.

According to this preferred embodiment of the invention, a foil diaphragm is used to close the hollow plunger barrel. Thereby the plunger may be used for making a chamber inside the syringe body, bounded by the inside surface of the syringe body barrel and the foil diaphragm. The foil diaphragm may be made from different materials, eg metal, plastic, or any other suitable material. The thickness of the foil diaphragm is preferably strong enough not to break by the pressure from the liquid when the plunger is depressed, and thin enough to easily let the sharp edge of the latch or needle mount perforate it. The foil diaphragm is preferably glued or welded to the plunger barrel, but other means of fixation may equally well be used within the scope of the invention.

When said plunger (2) comprises a thickening (23), an advantageous embodiment of the present invention has been obtained.

According to this preferred embodiment of the present invention, the plunger comprises a thickening precisely adapted to fill the syringe barrel. Thereby a fluid tight chamber may be made inside the syringe barrel.

When the end of said latch (17) is sharp, an advantageous embodiment of the present invention has been obtained.

According to this preferred embodiment of the invention, the upper end of the latch is sharp, and thereby able to perforate the foil diaphragm of the plunger.

When said latch (17) has an inside shape (19) corresponding to the outside shape (33) of said needle mount (32), an advantageous embodiment of the present invention has been obtained.

According to this preferred embodiment of the invention, the shape of the latch is adapted to correspond to the shape of the needle mount, and is thereby better suited to hold the needle mount firmly.

When said latch (17) completely surrounds said needle mount (32), an advantageous embodiment of the present invention has been obtained.

According to this preferred embodiment of the invention, the sharp end of the latch makes a full circle, and is thereby more suited to cut out the middle piece of the foil diaphragm of the plunger.

When said latch (17) has at least two vertical slits (20), an advantageous embodiment of the present invention has been obtained.

According to this preferred embodiment of the invention, the latch is able to bend outwards to release the needle mount, even when it fully surrounds the needle mount.

When said outside shape (33) of said needle mount (32) is the shape of a spherical segment, an advantageous embodiment of the present invention has been obtained.

According to this preferred embodiment of the invention, the needle mount may be ball shaped, and the fixation means thereby be a ball catch.

When said cavity (34) is concave, an advantageous embodiment of the present invention has been obtained.

According to this preferred embodiment of the invention, possible air bubbles in the liquid drawn into the syringe may easily be collected in the cavity, and subsequently pushed out of the syringe prior to injection.

When said cavity (34) has the shape of a spherical segment, an advantageous embodiment of the present invention has been obtained.

When the edge of said cavity (34) is sharp, an advantageous embodiment of the present invention has been obtained.

According to this alternative embodiment of the present invention, the edge of the cavity is sharpened, and thereby able to perforate the foil diaphragm. If this embodiment of the present invention is used, the end of the latch may be dull, as the foil diaphragm is then cut off by the cavity edge.

When said syringe body (1) comprises a spring housing (14), an advantageous embodiment of the present invention has been obtained.

When said hypodermic needle (31) is spring loaded by means of a spring (4) positioned in said spring housing (14), an advantageous embodiment of the present invention has been obtained.

When the length of said spring (4), when compressed, is adapted to the distance between the external end of said spring housing (14) and the needle mount (32) when retained by said fixation means (16), an advantageous embodiment of the present invention has been obtained.

According to this preferred embodiment of the invention, the length of the spring is at least partly chosen on the basis of the distance between the bottom of the spring housing and the bottom of the retained needle mount. When the needle mount is in this position, the spring must be compressed and engaging the needle mount with a force sufficient to cause the latch to bend outwards if the locking ring is removed.

When the length of said spring (4), when loose, is adapted to ensure retraction of the full length of the needle (31), an advantageous embodiment of the present invention has been obtained.

According to this preferred embodiment of the invention, the length of the spring is at least partly chosen on the basis of the distance between the bottom of the spring housing and the bottom of the needle mount, when it is in a fully retracted position, i.e. when the sharp end of the needle is fully retracted into the syringe.

When said syringe body (1) comprises at least one inwardly projecting part (13), an advantageous embodiment of the present invention has been obtained.

According to this preferred embodiment of the present invention, it is not possible to remove the plunger from the syringe body without using excessive force, as the inwardly projecting bulges, or alternatively a bulging ring, makes the smallest inside diameter of the syringe barrel smaller than the biggest outside diameter of the plunger, represented by the thickening. Thereby it is impossible to dismantle the syringe, and thereby expose the used and possibly disease carrying needle, when the release mechanism first has been used, and the needle is retracted into the barrel.

### List of drawings

The invention is in the following described with reference to the drawings, of which
fig. 1 shows an exploded view of an embodiment of the invention,
fig. 2 shows an embodiment of the invention as it looks before use,
fig. 3 shows an embodiment as it looks during an injection,
fig. 4 shows an embodiment when the plunger has just been fully depressed,
fig. 5 shows an embodiment immediately after the plunger has been fully depressed,
fig. 6 shows an embodiment after the needle has been fully released and irreversibly located inside the hollow plunger,
fig. 7a-7b shows different embodiments of the cavity in the needle mount, and
fig. 8a-8c shows different embodiments of the needle mount.

### Detailed description

The hypodermic syringe of the present invention is used for giving injections to human beings or animals in the same way as other commonly known syringes. Thus fluid medicine or any other liquid is drawn into a chamber of the syringe by pulling its plunger, and the medicine or liquid is injected by letting the needle penetrate the tissue and preferably a vein of the patient, subsequently depressing the plunger, and thereby pressing the liquid from the chamber of the syringe into the tissue or vein of the patient.

When the plunger is fully depressed, it may be pushed an additional small distance into the syringe barrel, and thereby activate a needle release mechanism, causing the needle to be retracted into the syringe, and thereby effectively prevent further use of the syringe and also remove the risk of anyone sticking themselves on a used syringe needle.

Preferred embodiments of the present invention are in the following described in more detail.

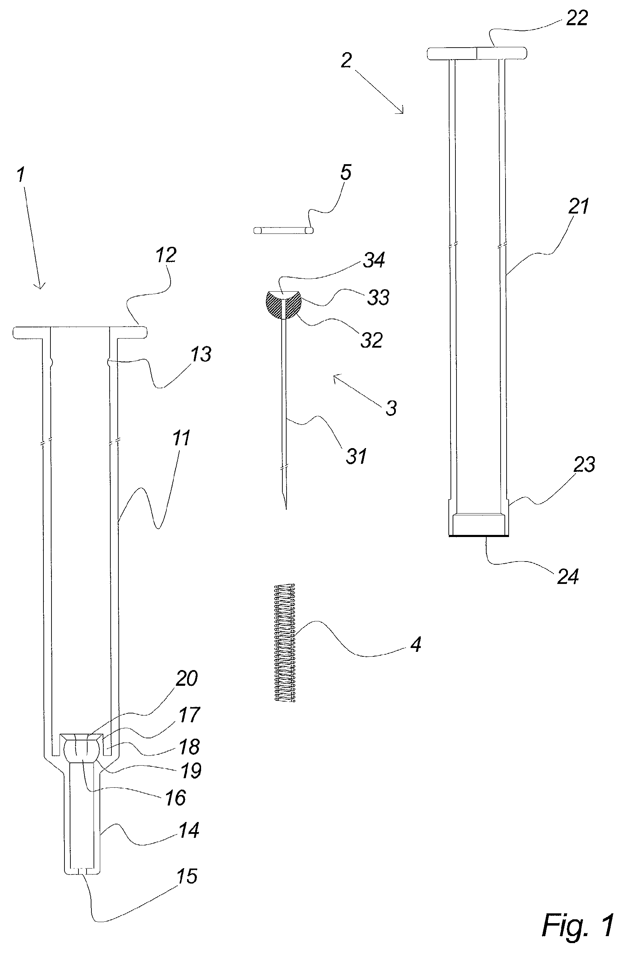

Figure 1 shows an exploded view of a preferred embodiment of a hypodermic syringe according to the present invention. It comprises a syringe body 1, a plunger 2, a hypodermic needle 3, a spring 4 and a locking ring 5.

The syringe body 1 is preferably made of a plastic material, preferably polypropylene, polysulfone, polystyrene, polyethylene, polycarbonate, polymethylmethacrylate or polyphenylsulfone, or any other plastic material, but may as well be made of other suitable materials, eg glass. It comprises a barrel 11, which in one end, the rear end, has an outside finger grip 12 and a number of inside bulges 13, or alternatively a bulging ring, and in the opposite end, the front end, has fixation means 16. A latch 17 made of a relatively soft material, eg plastic, and preferably the same material as the syringe body 1, forms the fixation means 16. Between the latch 17 and the inside surface of the barrel 11 is a recess 18. In elongation of the barrel 11 is a spring housing 14, having a bore 15. In a preferred embodiment the latch 17 describes a full circle, and has then at least two slits 20. Other embodiments comprise broader slits or a parting of the latch in eg 3 narrow parts. The latch 17 has an inside surface 19.

The plunger 2 is preferably made of a plastic material, preferably the same material as the syringe body, eg polypropylene, polysulfone, polystyrene, polyethylene, polycarbonate, polymethylmethacrylate or polyphenylsulfone, etc., but may as well be made of any other suitable material, eg glass. It comprises a plunger barrel 21, which in one end, the external end, is closed by a finger grip 22, and in the opposite end, the internal end, is closed by a foil diaphragm 24. The latter end of the barrel 22 further comprises a thickening 23, which preferably is a silicone or rubber coating or band, but may be made of any suitable material, including the material of the plunger barrel 21 itself. The foil diaphragm may be made of materials such as metal or plastic, or other suitable material. The foil diaphragm should be strong enough to not break on the pressure applied to push liquid out of the syringe when injecting, but weak enough to be easily perforated by the sharp edge of the latch or the cavity.

The hypodermic needle 3 comprises a hollow needle 31 with a dull internal end and a sharpened external end, whose internal end is mounted in a needle mount 32. The needle mount may be made of plastic, rubber, metal or other suitable materials, and has a cavity extending from its internal end.

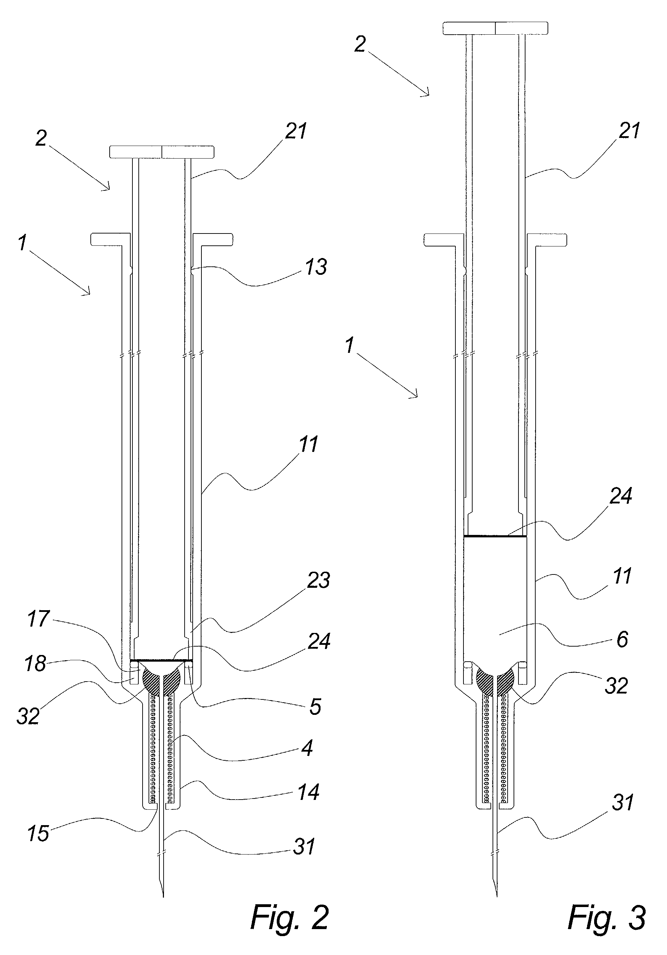

Fig. 2 shows an embodiment of the invention as it looks before use. The spring 4 is located in the spring housing 14, and the needle mount 32 is located in the fixation means 16, retained by the latch 17. To prevent the latch 17 from releasing the needle mount 32, the locking ring 5 is placed in the recess 18 around the top of the latch 17, leaving a substantially depth of the recess 18 empty beneath it. The needle 31 is extending out from the spring housing 14 through the bore 15. The lengths of the spring housing 14 and the spring 4 are selected to ensure compression of the spring when it is situated between the bore end of the spring housing 14 and the needle mount 32.

The plunger barrel 21 is positioned inside the barrel 11 of the syringe body 1, in such a way that the external end of the plunger barrel 21 extends from the rear end of the syringe barrel 11. The thickening 23 of the plunger barrel 21 is dimensioned to precisely fit the inner surface of the syringe barrel 11, and accordingly the bulges 13, or alternatively the bulging ring, are dimensioned to support the plunger barrel 11. Furthermore the bulges 13 prevents the plunger 2 from being totally pulled out of the syringe barrel 11, as the outer diameter of the thickening 23 is greater than the inside diameter of the bulging part of the syringe barrel 11.

Figure 3 shows an embodiment of the present invention, as it looks during an injection. The plunger 2 is partly pulled out of the syringe barrel 11, thus forming a chamber 6, surrounded by the foil diaphragm 24, the inside surface of the syringe barrel 11, and the needle mount 32. As the hollow needle 31 extends all the way through the needle mount 32, thus penetrating it, the chamber may be filled or emptied through the hollow needle 31 by pulling or pushing the plunger 2 accordingly.

During the injection, when the plunger barrel 21 with the foil diaphragm 24 reaches and rests on the locking ring 5, the situation will look the same as shown in figure 2, described above. Due to the locking ring 5, the user pushing the plunger 2 will feel a certain resistance, but is with a little additional pressure able to fully depress the plunger 2, by pressing the locking ring 5 down to the bottom of the recess 18.

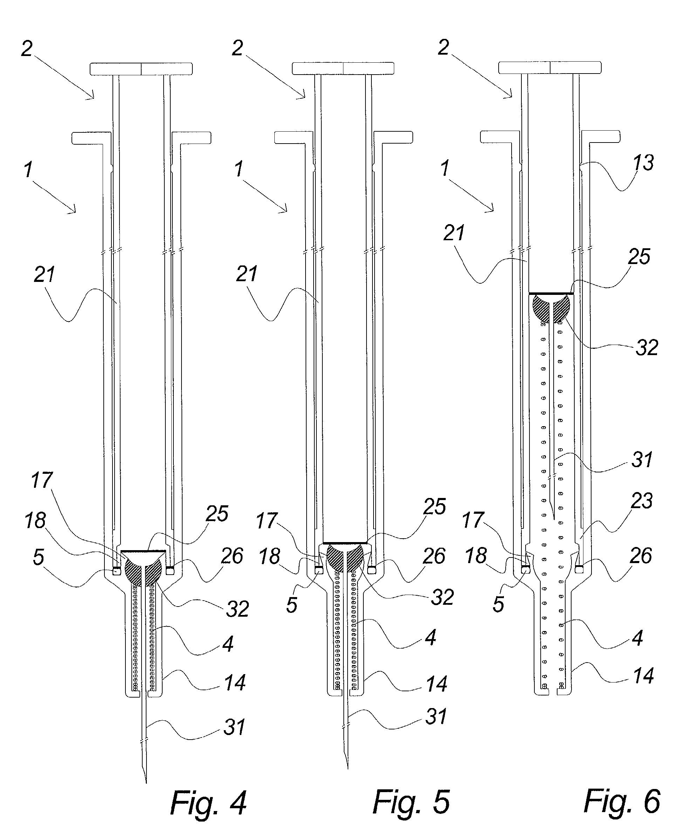

Figure 4 shows an embodiment of the present invention, when the plunger 2 has just been fully depressed. The plunger barrel 21 has pushed the locking ring 5 into the bottom of the recess 18. Thereby the thin foil diaphragm 24 is perforated by the sharp edge of the latch 17 and broken into two parts, whereof a rim 26 is still positioned at the end of the plunger barrel 21, and the middle part 25 is cut free and laying on top of the latch 17 and needle mount 32.

Figure 5 shows an embodiment of the present invention, immediately after the plunger has been fully depressed as shown in figure 4. Due to the removal of the locking ring 5 from the top of the latch 17, it is no longer able to retain its grip on the needle mount 32 biased by the spring 4, and it bends slightly outwards into the recess 18, and thereby releases the needle mount 32. The cut-off part 25 of the foil diaphragm 24 is resting on top of the needle mount 32, and thus following its movement.

Figure 6 shows an embodiment of the present invention, after the needle has been fully released and irreversibly located inside the hollow plunger. In continuation of the situation of figure 5, the spring 4 is now fully uncompressed, causing the needle mount 32 with the needle 31 to be retained inside the hollow plunger barrel 21. The length of the uncompressed spring 4 is selected according to the full length of the needle 31, so a full retraction of the needle to the inside of the syringe is ensured. In a preferred embodiment of the invention, the bulge 13 and the thickening 23 effectively prevents the plunger 2 from being removed from the syringe body 1 without use of excessive force. Thereby is ensured that the used and possibly disease carrying needle is not again exposed.

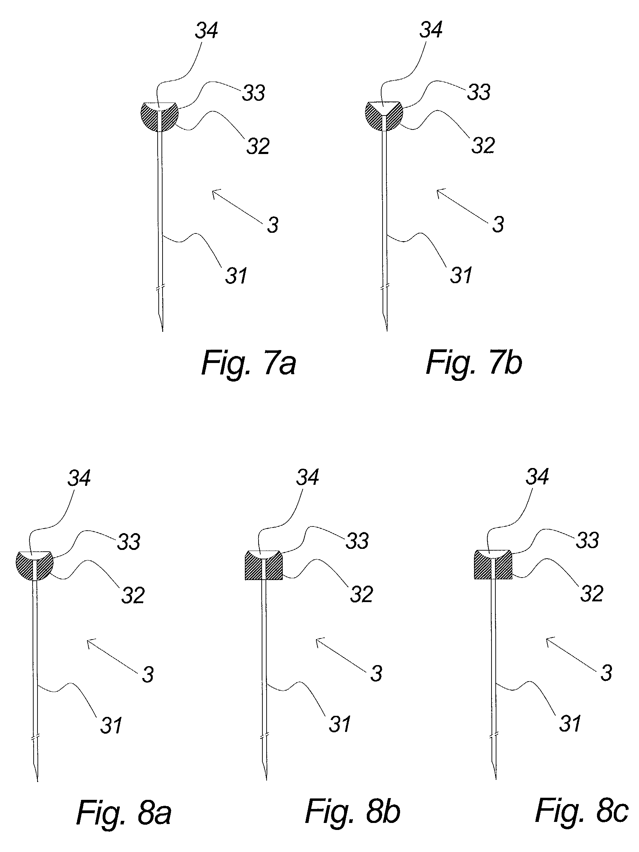

Figure 7a-7b shows different embodiments of the needle mount 32. Figure 7a shows a preferred embodiment of a needle mount 32 with a cavity 34 having the shape of a spherical segment. Figure 7b shows a needle mount 32 with a cavity having the shape of a truncated pyramid or a truncated cone. Other shapes of the cavity may be applied within the scope of the present invention. Any shape that facilitates the collection of air bubbles when the syringe is rotated so the needle points upward, is suitable.

Figure 8a-8c shows different embodiment of the needle mount 32. Figure 8a shows a preferred embodiment of a needle mount 32 having an outside shape 33 being the shape of a spherical segment. Figure 8b shows a needle mount 32 having an outside shape 33 that is angular. Figure 8c shows a needle mount 32 having an outside shape 33 that is hyperbolic. Other shapes of the needle mount may be applied within the scope of the present invention. Any shape that enables a correspondingly formed latch to get a hold on it is suitable.

## Claims

1. A disposable hypodermic syringe comprising
a syringe body (1), comprising fixation means (16) for releasably retaining a spring loaded hypodermic needle (31) whose internal end is mounted in a needle mount (32), and a plunger (2);
**characterized in that**
said needle mount (32) has a cavity (34) extending from its internal end.

2. A disposable hypodermic syringe according to claim 1, wherein said fixation means (16) comprises a latch (17), a locking ring (5) and a recess (18).

3. A disposable hypodermic syringe according to claim 1 or 2, wherein said plunger (2) comprises a hollow barrel (21).

4. A disposable hypodermic syringe according to claim 3, wherein the internal end of said hollow barrel (21) of said plunger (2) is closed with a foil diaphragm (24).

5. A disposable hypodermic syringe according to any of the claims 1 to 4, wherein said plunger (2) comprises a thickening (23).

6. A disposable hypodermic syringe according to claim 2, wherein the end of said latch (17) is sharp.

7. A disposable hypodermic syringe according to claim 2 or 6, wherein said latch (17) has an inside shape (19) corresponding to the outside shape (33) of said needle mount (32).

8. A disposable hypodermic syringe according to claim 2, 6 or 7, wherein said latch (17) completely surrounds said needle mount (32).

9. A disposable hypodermic syringe according to any of the claims 2 or 6 to 8, wherein said latch (17) has at least two vertical slits (20).

10. A disposable hypodermic syringe according to claim 1 or 7, wherein said outside shape (33) of said needle mount (32) is the shape of a spherical segment.

11. A disposable hypodermic syringe according to any of the claims 1 to 10, wherein said cavity (34) is concave.

12. A disposable hypodermic syringe according to any of the claims 1 to 11, wherein said cavity (34) has the shape of a spherical segment.

13. A disposable hypodermic syringe according to any of the claims 1 to 12, wherein the edge of said cavity (34) is sharp.

14. A disposable hypodermic syringe according to claim 1, wherein said syringe body (1) comprises a spring housing (14).

15. A disposable hypodermic syringe according to claim 14, wherein said hypodermic needle (31) is spring loaded by means of a spring (4) positioned in said spring housing (14).

16. A disposable hypodermic syringe according to claim 15, wherein the length of said spring (4), when compressed, is adapted to the distance between the external end of said spring housing (14) and the needle mount (32) when retained by said fixation means (16).

17. A disposable hypodermic syringe according to claim 15 or 16, wherein the length of said spring (4), when loose, is adapted to ensure retraction of the full length of the needle (31).

18. A disposable hypodermic syringe according to claim 1 or 5, wherein said syringe body (1) comprises at least one inwardly projecting part (13).
